# EUROPEAN PATENT APPLICATION

(11) **EP 4 692 340 A1**
(43) Date of publication of application: **11.02.2026**
(21) Application number: 24823725.7
(22) Date of filing: 13.06.2024
(51) Int. Cl.: C12N 9/52, C12N 15/75, A23K 10/16

(54) **NOVEL SERINE PROTEASE VARIANT**

(30) Priority: 13.06.2023 KR 20230075589
(71) Applicant: CJ Cheiljedang Corporation, Seoul 04560 (KR)
(72) Inventor: LEE, Joo Hee, Seoul 04560 (KR); JI, Chang Jun, Seoul 04560 (KR); SEOK, Jong-cheol, Seoul 04560 (KR); CHO, A-Ra, Seoul 04560 (KR)
(74) Representative: Meissner Bolte Partnerschaft mbB
(86) International application number: PCT/KR2024/008126
(87) International publication number: WO 2024/258208

(57) **Abstract**

The present disclosure relates to a novel serine protease variant.

## Description

### [Technical Field]

The present disclosure relates to a novel serine protease variant.

### [Background Art]

Proteases are involved in various functions such as digestion, absorption, and defense in living organisms, and are classified into serine proteases, cysteine proteases, aspartic proteases, and metalloproteases according to the structures of active sites. Among them, serine proteases (or serine endopeptidases) are enzymes characterized by having in common an active serine residue in their active sites, which cleave peptide bonds in proteins, in which serine serves as a nucleophilic amino acid at a protease's active site (Hedstrom, 2002. Chem Rev 102: 4501-4524).

Serine proteases have been used in a wide variety of applications. In addition to therapeutic applications to treat human diseases such as lysis of blood clots, serine proteases are used not only as ingredients of laundry detergents and contact lens cleaners, but also in modification of milk proteins, silk degumming, soaking of leather, unhairing, synthesis of oligopeptides, recovery of silver from lung X-ray films, production and improvement of feeds and foods, and the like. However,

### [Disclosure]

### [Technical Problem]

In order to secure greater industrial economic feasibility and efficacy, a serine protease improved in terms of thermal stability, activity, etc. is required.

### [Technical Solution]

An aspect of the present disclosure provides a serine protease variant, in which an amino acid corresponding to position 180 of SEQ ID NO: 10 is substituted with a different amino acid.

In one specific embodiment, the different amino acid may be tyrosine (Y), glutamic acid (E), histidine (H), leucine (L), methionine (M), asparagine (N), glutamine (Q), or tryptophan (W).

In any one specific embodiment of the previous specific embodiments, the serine protease variant may comprise an amino acid sequence having 75% or more sequence homology or identity to SEQ ID NO: 10.

In any one specific embodiment of the previous specific embodiments, the serine protease variant may comprise an amino acid sequence having 75% or more sequence homology or identity to SEQ ID NO: 7.

In any one specific embodiment of the previous specific embodiments, the serine protease variant may be a serine protease variant, in which an amino acid corresponding to position 331 of SEQ ID NO: 9 or SEQ ID NO: 12 is substituted with a different amino acid.

In any one specific embodiment of the previous specific embodiments, the serine protease variant may be a serine protease variant, in which an amino acid corresponding to position 361 of SEQ ID NO: 8 or SEQ ID NO: 11 is substituted with a different amino acid.

Another aspect of the present disclosure provides a polynucleotide encoding the serine protease variant.

Still another aspect of the present disclosure provides a vector comprising the polynucleotide.

Still another aspect of the present disclosure provides a microorganism comprising the serine protease variant, the polynucleotide encoding the variant, or the vector comprising the polynucleotide.

In one specific embodiment, the microorganism may be a microorganism of the genus *Bacillus (Bacillus* sp.). In any one specific embodiment of the previous specific embodiments, the microorganism may be *Bacillus subtilis.*

Still another aspect of the present disclosure provides a composition comprising any one or more of the serine protease variant; and the microorganism comprising the serine protease variant, the polynucleotide encoding the variant, or the vector comprising the polynucleotide.

In one specific embodiment, the composition may be a feed composition.

Still another aspect of the present disclosure provides a method of preparing the serine protease variant, the method comprising the step of culturing the microorganism comprising the serine protease variant, the polynucleotide encoding the variant, or the vector comprising the polynucleotide.

In one specific embodiment, the method of preparing the serine protease variant may further comprise the step of collecting the variant polypeptide having the serine protease activity of the present disclosure, which is expressed in the culturing step.

Still another aspect of the present disclosure provides a method of degrading casein using the serine protease variant.

### [Advantageous Effects]

A serine protease variant of the present disclosure has superior activity compared to the existing serine protease, thereby having useful industrial applications.

### [Detailed Description of the Invention]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

Further, a number of papers and patent documents are referenced and cited throughout this specification. The disclosures of the cited papers and patent documents are incorporated herein by reference in their entirety to further clarify the level and scope of the subject matter to which the present disclosure pertains.

### Definition

As used in the specification and appended claims of the present disclosure, the singular forms ("a", "an", and "the") comprise plural referents unless the context clearly states otherwise. Unless the context indicates otherwise, singular terms shall comprise the plural and plural terms shall comprise the singular. As used in the specification and appended claims of the present disclosure, unless stated otherwise, the use of "or" may be used to comprise "and/or".

As used herein, the term "consisting essentially of" may mean that, in the case where the features of the object claimed herein are not substantially affected by the presence of an unspecified component, the unspecified component may be present.

As used herein, the term, "consisting of" means that the total ratio of specific component(s) is 100%. The components or features that are recited after the term "consisting of" may be essential or mandatory. In some embodiments, in addition to the components or features recited after the term "consisting of", any other components or non-essential components may be excluded.

As used herein, the term "comprising" means the presence of features, steps or components recited after the term, and does not exclude the presence or addition of one or more features, steps or components. The components or features recited after the term "comprising" herein may be essential or mandatory. However, in some embodiments, the term may further comprise any other or non-essential components or features.

### Variant

As used herein, the term "protein" or "polypeptide" refers to a polymer or oligomer of consecutive amino acid residues. In the present disclosure, "polypeptide," "protein," and "peptide" may be used interchangeably with "amino acid sequence."

In some cases, an amino acid sequence exhibiting activity may be referred to as an "enzyme". In the present disclosure, unless indicated otherwise, amino acid sequences are described in an N-terminal to C-terminal direction.

As used herein, the term "mature polypeptide" means a polypeptide in a form without a signal sequence or propeptide sequence. A mature protein/polypeptide/peptide may be a functional form of a protein/polypeptide/peptide. The mature polypeptide may be in a final form after translation or post-translational modification. Examples of the posttranslational modification comprise N- or C-terminal modifications, glycosylation, phosphorylation, leader sequence removal etc., but are not limited thereto.

As used herein, the term "wild-type" refers to a naturally-occurring state without artificial modification. When the term "wild-type" is used in reference to a polypeptide, it refers to a naturally occurring polypeptide, which does not have artificial mutations (substitutions, insertions, deletions, etc.) in one or more amino acid positions. Similarly, when the term "wild-type" is used in reference to a polynucleotide, it means not having artificial modifications (substitutions, insertions, deletions) in one or more nucleotides. However, polynucleotides encoding wild-type polypeptides are not limited to wild-type polynucleotides, and comprise sequences encoding any wild-type polypeptides.

As used herein, the parent sequence or backbone refers to a reference sequence in which modification is introduced to be a variant. That is, the parent sequence may be served as a starting sequence in which modification such as substitutions, insertions, and/or deletions may be introduced. The parent sequence may be naturally-occurring or wild-type, or a variant in which one or more substitutions, insertions or deletions have occurred in the natural or wild type, or may be an artificially synthesized sequence. When the parent sequence is an amino acid sequence exhibiting activity, i.e., an amino acid sequence of an enzyme, it may be referred to as a parent enzyme.

As used herein, the term "reference sequence" means a sequence used to determine the position of amino acids within any amino acid sequence. Any amino acid sequence may be aligned with a reference sequence to determine the position of an amino acid that corresponds to a particular position in the reference sequence within any amino acid sequence.

As used herein, the term "position N" may comprise position N and an amino acid position corresponding to the position N, and specifically, may comprise an amino acid position corresponding to any amino acid residue in a mature polypeptide disclosed in a particular amino acid sequence.

As used herein, the term "corresponding to" refers to an amino acid residue at a position listed in a polypeptide, or an amino acid residue that is similar, identical or homologous to a residue listed in a polypeptide. Confirming the amino acid at the corresponding position may be determining a specific amino acid in a sequence that refers to a specific sequence. "Corresponding region" used herein generally refers to a similar or corresponding position in a related protein or reference protein.

In the present disclosure, SEQ ID NO: 10 may be used as a reference sequence to determine the position of an amino acid in any amino acid sequence.

In other words, SEQ ID NO: 10 disclosed in the present disclosure may be used to determine the corresponding amino acid residue in any polypeptide having the serine protease activity, and unless otherwise specified in the present disclosure, the residue of a specific amino acid sequence is numbered based on SEQ ID NO: 10.

For example, any amino acid sequence is aligned with SEQ ID NO: 10, and based on this, each amino acid residue of the amino acid sequence may be numbered by reference to the numerical position of the amino acid residue of SEQ ID NO: 10 and the corresponding amino acid residue. For example, a sequence alignment algorithm such as that described herein may confirm the position of an amino acid, or a position where a modification such as substitution, insertion or deletion occurs, compared to a query sequence (also referred to as a "reference sequence").

For such alignment, for example, Needleman-Wunsch algorithm (Needleman and Wunsch, 1970, J. Mol. Biol. 48: 443-453), Needle program of EMBOSS package (EMBOSS: The European Molecular Biology Open Software Suite, Rice et al., 2000), Trends Genet. 16: 276-277), and the like may be used without being limited thereto, and sequence alignment programs, pairwise sequence comparison algorithm, and the like known in the art may be appropriately used.

In addition, an amino acid residue corresponding to other polypeptide may be identified by multiple sequence alignment. Examples of the multiple sequence alignment program known in the art may comprise programs such as multiple sequence comparison by log-expectation (MUSCLE; version 3.5 or later; Edgar, 2004, Nucleic Acids Research 32: 1792-1797), MAFFT (version 6.857 or later; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537: 39-64; Katoh and Toh, 2010, Bioinformatics 26: 1899-1900), etc., and EMBOSS EMMA using ClustalW (1.83 or later; Thompson et al., 1994, Nucleic Acids Research 22 : 4673-4680), etc., and respective default parameters thereof may be used, but are not limited thereto.

In addition, when relationship between polypeptides derived from the mature polypeptide cannot be detected by conventional sequence-based comparison, other pairwise sequence comparison algorithms may be used (Lindahl and Elofsson, 2000, J. Mol. Biol. 295: 613-615). Higher sensitivity in sequence-based searching may be obtained using search programs using probabilistic representations of polypeptide families (profiles) to search database. For example, PSI-BLAST program generates profiles through an iterative database search process and may detect remote homologs (Atschul et al., 1997, Nucleic Acids Res. 25: 3389-3402). Even higher sensitivity may be obtained when the family or superfamily for the polypeptide has one or more representatives in the protein structure database. Programs such as GenTHREADER (Jones, 1999, J. Mol. Biol. 287: 797-815; McGuffin and Jones, 2003, Bioinformatics 19: 874-881) utilize information from a variety of sources, such as PSI-BLAST, secondary structure prediction, structural alignment profiles, and solvation potentials, as input to a neural network that predicts the structural folding for a query sequence. Similarly, a method introduced by Gough et al., 2000, J. Mol. Biol. 313: 903-919 may be used to align a sequence of an unknown structure with the superfamily models present in the SCOP database. These alignments may be used in turn to generate a homology model for the polypeptide, and the models may be assessed for accuracy using a variety of tools developed for that purpose.

For proteins of known structures, several tools and resources are available for retrieving and generating structural alignment. For example, the SCOP superfamilies of proteins have been structurally aligned, and those alignments are accessible and downloadable. Two or more protein structures may be aligned using a variety of algorithms such as the distance alignment matrix (Holm and Sander, 1998, Proteins 33: 88-96) or Combinatiorial extension (CE) Shindyalov and Bourne, 1998, Protein Engineering 11: 739-747). Implementation of these algorithms may additionally be utilized to query structure databases with a structure of interest in order to discover possible structural homologs (Holm and Park, 2000, Bioinformatics 16: 566-567).

The above-described methods are merely examples, and the present disclosure is not limited thereto.

With regard to an amino acid sequence in the present disclosure, although being described as a polypeptide "comprising" an amino acid sequence represented by a specific SEQ ID NO., a polypeptide "consisting of" an amino acid sequence represented by a specific SEQ ID NO., or a polypeptide or protein "having" an amino acid sequence represented by a specific SEQ ID NO., it is obvious that any protein having an amino acid sequence with deletion, modification, substitution, conservative substitution, or addition of some sequence may be comprised in the scope of the present disclosure as long as the protein has activity identical or corresponding to that of the protein consisting of the amino acid sequence of the corresponding SEQ ID NO. For example, as long as a protein has activity identical or corresponding to that of the variant protein, it does not exclude addition of a sequence, which does not alter the function of the protein, upstream or downstream of the amino acid sequence, a naturally occurring mutation, a silent mutation thereof, or conservative substitution, and it is obvious that such a sequence addition or mutation also falls within the scope of the present disclosure.

For example, there are cases of having addition of a sequence which does not alter the function of the variant polypeptide of the present disclosure at the N-terminus, C-terminus and/or within the amino acid sequence, a naturally occurring mutation, a silent mutation, or a conservative substitution.

As used herein, the term "conservative substitution" means substituting an amino acid with another amino acid having similar structural and/or chemical properties. Such amino acid substitution may generally occur based on similarity in the polarity, charge, solubility, hydrophobicity, hydrophilicity and/or amphipathic nature of the residue. Typically, the conservative substitution may have little or no effect on the activity of polypeptide.

As used herein, the term "another amino acid" is not limited as long as it is an amino acid different from the amino acid before substitution. Meanwhile, when it is expressed that "a specific amino acid has been substituted", it is obvious that the amino acid is substituted with an amino acid different from the amino acid before substitution, even though it is not specifically stated that the amino acid has been substituted with a different amino acid.

In the present disclosure, the "varying/modifying" refers to changing or altering. This may be alteration from naturally occurring. For example, a polypeptide may be changed by altering the parent sequence or reference sequence of the polypeptide.

In the present disclosure, the modified polypeptide may be a polypeptide not existing in nature, i.e., a polypeptide which does not occur naturally.

As used herein, the term "modified" refers to, for example, altered from a naturally occurring form. A modified polypeptide of the present disclosure comprises a polypeptide which does not occur naturally or a naturally occurring variant. For example, the modified polypeptide of the present disclosure is a modified polypeptide not found in nature. For example, the modified polypeptide of the present disclosure may not be a spontaneously occurring polypeptide, but is not limited thereto.

When used in connection with an amino acid/nucleic acid sequence, the term "modification" used herein may comprise substitution of an amino acid/nucleic acid residue at one or more site of a parent sequence with a different amino acid/nucleic acid residue, deletion of an amino acid/nucleic acid residue (or a series of amino acid/nucleic acid residues) of the parent sequence at one or more sites, insertion of an amino acid/nucleic acid residue (or a series of amino acid/nucleic acid residues) into the parent sequence at one or more sites, truncation of an amino acid sequence of the N-terminus and/or the C-terminus or a 5' and/or 3' nucleic acid sequence, and any combination thereof.

In the present disclosure, the "variant" or "variant polypeptide" refers to a polypeptide in which one or more amino acids are conservatively substituted and/or modified so as to be different from the polypeptide of the parent sequence, while retaining functions or properties of the polypeptide. The variant polypeptide differs from an identified sequence by substitution, deletion, or addition of several amino acids. Such a variant polypeptide may generally be identified by modifying one or more amino acids in the amino acid sequence of the polypeptide and evaluating the properties of the modified polypeptide. In other words, the ability of the variant polypeptide may be increased, unchanged, or decreased, compared to the native polypeptide. Further, some variant polypeptides may comprise variant polypeptides in which one or more portions, such as an N-terminal leader sequence or transmembrane domain, have been removed. Other variant polypeptides may comprise variants in which a portion has been removed from the N- and/or C-terminus of a mature protein. The term "variant polypeptide" may also be used as a modification, modified protein, mutant, mutein, divergent, variant, etc., and any term is not limited, as long as it is used in a sense of being mutated.

Further, the variant polypeptide may also comprise deletion or addition of amino acids that have minimal influence on the properties and secondary structure of the polypeptide. For example, a polypeptide may be conjugated to a signal (or leader) sequence at the N-terminus of the polypeptide, which co-translationally or post-translationally directs transfer of the protein. The polypeptide may also be conjugated to other sequence or a linker for identification, purification, or synthesis of the polypeptide.

The serine protease variant of the present disclosure may be in an isolated form.

As used herein, the term "isolated" refers to a substance present in an environment which does not occur naturally or present in a form which does not exist naturally. This comprises that a substance (sequence or nucleic acid) is at least substantially free from at least other components having the substance, e.g., sequence or nucleic acid, which is naturally associated in nature and as found in nature.

For example, the isolated sequence or nucleic acid provided in the present disclosure may be provided in a form that is substantially free of one or more contaminants.

Examples of the isolated substance may comprise i) any substance which is not naturally occurring, ii) any substance from which one or more, or all naturally-occurring component associated in nature are removed (e.g., enzyme, variant, nucleic acid, protein, peptide, or cofactor), iii) any substance that has been artificially modified from a substance found in nature, or iv) a substance modified to change the amount of the substance compared to other components naturally associated therewith (e.g., by increasing the copy number of a gene encoding a particular substance; by modifying a promoter naturally associated with a gene encoding a particular substance with a promoter having stronger activity, etc.), but are not limited thereto.

In the present discourse, in relation to an amino acid or nucleic acid sequence, the term "biologically active fragments or fragments" may refer to "functional fragments". The "functional fragment" may also refer to an active fragment, and refers to a polypeptide that comprises fewer amino acids than a full-length protein but possesses at least one biological activity of the corresponding full-length protein. For example, the functional fragment of the enzyme may comprise a catalytic site of the enzyme.

The serine protease variant of the present disclosure may comprise biologically active fragments of the serine protease variant. The biologically active fragments may comprise a part of the full length of a native polypeptide.

For example, the fragment may comprise at least about 20%, 30%, 40%, 50%, 60%, 70%, 80%, 90%, 95%, 98%, 99% or more, and less than 100% of amino acids of the full length of the native polypeptide, but is not limited thereto.

Throughout the specification, the conventional one-letter and three-letter codes for naturally occurring amino acids are used. In addition, the amino acids mentioned herein are abbreviated according to the nomenclature rules of IUPAC-IUB.

| | |
|---|---|
| alanine Ala, A | arginine Arg, R |
| asparagine Asn, N | aspartic acid Asp, D |
| cysteine Cys, C | glutamic acid Glu, E |
| glutamine Gln, Q | glycine Gly, G |
| histidine His, H | isoleucine Ile, I |
| leucine Leu, L | lysine Lys, K |
| methionine Met, M | phenylalanine Phe, F |
| proline Pro, P | serine Ser, S |
| threonine Thr, T | tryptophan Trp, W |
| tyrosine Tyr, Y | valine Val, V |

Meanwhile, any amino acid may be described as Xaa, X.

Also, three-letter codes generally allowed for other amino acids, such as 2-aminoisobutyric acid (Aib), N-methylglycine (Sar), and α-methyl-glutamic acid, may be used as well as the naturally occurring amino acids.

Amino acids may generally be classified based on similarity of polarity, charge, solubility, hydrophobicity, hydrophilicity, and/or amphipathic nature of residues.

Examples of this classification may comprise positively charged (basic) amino acids such as arginine, lysine, and histidine; negatively charged (acidic) amino acids such as glutamic acid and aspartic acid; amino acids with nonpolar side chains (nonpolar amino acids) such as glycine, alanine, valine, leucine, isoleucine, methionine, phenylalanine, tryptophan, and proline; amino acids with polar or hydrophilic side chains (polar amino acids) such as serine, threonine, cysteine, tyrosine, asparagine, and glutamine. For another example, amino acids may be classified into amino acids with electrically charged side chains (electrically charged amino acids) such as arginine, lysine, histidine, glutamic acid, and aspartic acid, and amino acids with uncharged side chains (uncharged amino acids; also called neutral amino acids) such as glycine, alanine, valine, leucine, isoleucine, methionine, phenylalanine, tryptophan, proline, serine, threonine, cysteine, tyrosine, asparagine, and glutamine. For still another example, phenylalanine, tryptophan, and tyrosine may be classified as aromatic amino acids. For still another example, valine, leucine, and isoleucine may be classified as branched amino acids. For still another example, 20 types of amino acids are classified according to size, starting from the amino acid group with relatively small volume, the amino acids may be classified into five groups; glycine, alanine, serine; cysteine, proline, threonine, aspartic acid, asparagine; valine, histidine, glutamic acid, glutamine; isoleucine, leucine, methionine, lysine, arginine; and phenylalanine, tryptophan, tyrosine. However, they are not necessarily limited thereto.

In order to describe the variant provided in the present disclosure, the following nomenclature is used.

In the present disclosure, referring to a specific position in an amino acid sequence may comprise referring to an amino acid present or substituted at that position. Referring to an amino acid at a specific position may be described in various ways. For example, the "position 003" may be described as "position 3", "amino acid 3", "3^{rd} amino acid". Further, for example, when the amino acid at position 3 is serine (S), it may be described as "S3" or "Ser3".

Amino acid substitution may be expressed by describing the amino acid before substitution, the position, and the amino acid to be substituted in sequence. The amino acids may be expressed using the conventional one-letter and three-letter codes. For example, when alanine, an amino acid at position 6 of a specific sequence, is substituted with valine, it may be described as "A6V" or "Ala6Val".

Any amino acid at a specific position may be referred to as "X". For example, X6 refers to any amino acid at position 6. In addition, when the amino acid to be substituted is expressed as X, it means that the amino acid is substituted with an amino acid different from the amino acid present before substitution. For example, "V6X" indicates that V at position 6 is substituted with any amino acid other than V.

### Polynucleotide

As used herein, the term "gene" means a polynucleotide that encodes a polypeptide and a polynucleotide comprising regions the upstream and downstream of the coding region. In some embodiments, a gene may have a sequence (intron) inserted between respective coding regions (exons).

As used herein, the term "polynucleotide, nucleic acid, or nucleic acid molecule" refers to a DNA (e.g., cDNA or genomic DNA) or RNA strand of a certain length or longer as a polymer of nucleotides in which nucleotide monomers are connected in a long chain form by covalent bonds.

### Homology, Identity

As used herein, the term "homology" or "identity" refers to a degree of relevance between two given amino acid sequences or nucleotide sequences, and may be expressed as a percentage. The terms homology and identity may often be used interchangeably with each other.

The sequence homology or identity of a conserved polynucleotide or polypeptide is determined by standard alignment algorithms, and the default gap penalty established by a program used may be used together. Substantially, homologous or identical sequences may generally hybridize under moderately or highly stringent conditions to the entirety of the sequence or at least about 50%, 60%, 70%, 80%, or 90% of the full-length. It is apparent that hybridization also comprises hybridization of a polynucleotide with a polynucleotide including a general codon or a codon in consideration of codon degeneracy.

Whether or not any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined using Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (comprising GCG program package ((Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO et al.](1988) SIAM J Applied Math 48: 1073). For example, BLAST of the National Center for Biotechnology Information or ClustalW may be used to determine the homology, similarity, or identity.

The homology, similarity, or identity between polynucleotides or polypeptides may be determined by comparing sequence information using the GAP computer program as introduced by, for example, Needleman et al. (1970), J Mol Biol. 48:443, as disclosed by Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. The default parameters for the GAP program may comprise: (1) unitary matrices (containing a value 1 for identity and a value 0 for non-identity), PAM Matrix (see those described by Schwartz and Dayhoff, eds., Atlas Of Protein Sequence And Structure, National Biomedical Research Foundation (1978)), and the weighted comparison matrix of Gribskov et al(1986) Nucl. Acids Res. 14: 6745 (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps.

Further, whether any two polynucleotide or polypeptide sequences have a homology, similarity or identity with each other may be identified by comparing the sequences in a Southern hybridization experiment under stringent conditions as defined, and appropriate hybridization conditions defined are within the skill of the art, and may be determined by a method well known to those skilled in the art (e.g., J. Sambrook et al., Molecular Cloning, A Laboratory Manual; F.M. Ausubel et al., Current Protocols in Molecular Biology, John Wiley & Sons, Inc., New York), but is not limited thereto.

As used herein, the term "stringent conditions" mean conditions that enable specific hybridization between polynucleotides. These conditions are specifically described in documents (see Sambrook et al., supra, 9.50-9.51, 11.7-11.8). Examples thereof comprise a condition in which polynucleotides having higher homology or identity, i.e., polynucleotides having 60% or more, 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed once, specifically, twice to three times at a salt concentration and temperature equivalent to 60°C, 1XSSC, 0.1% SDS, specifically 60°C, 0.1XSSC, 0.1% SDS, more specifically, 68°C, 0.1XSSC, 0.1% SDS, which are washing conditions for common Southern hybridization.

The hybridization requires that two nucleotides have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also comprise substantially similar nucleic acid sequences as well as isolated nucleic acid fragments that are complementary to the entire sequence.

The appropriate stringency to hybridize the polynucleotide depends on the length and degree of complementarity of the polynucleotide, and the variables are well known in the art (e.g., J. Sambrook et al., supra).

### Nucleic acid construct, Vector, Transformation

As used herein, the term "nucleic acid construct" refers to a single- or doublestranded nucleic acid molecule, which comprises one or more regulatory sequences, and which is artificially synthesized, or engineered to comprise a specific sequence in a manner that does not exist in nature, or isolated from nature.

As used herein, the term "vector" may comprise a DNA construct comprising a nucleotide sequence of a polynucleotide encoding a polypeptide of interest which is operably linked to a suitable expression regulatory region (or expression control sequence) so that the polypeptide of interest may be expressed in a suitable host. The expression regulatory region may comprise a promoter capable of initiating transcription, any operator sequence for controlling the transcription, a sequence encoding a suitable mRNA ribosome binding site, and a sequence controlling termination of transcription and translation. The vector may be transformed into a suitable microorganism, and then replicated or function independently of the host genome, or may be integrated into the genome itself.

The vector used in the present disclosure is not particularly limited, but any vector known in the art may be used. Examples of commonly used vectors comprise natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDZ system, pDC system, pBR system, pUC system, pBluescript II system, pGEM system, pTZ system, pCL system, pET system, etc. may be used as a plasmid vector. For example, pDZ, pDC, pACYC177, pACYC184, pCL, pECCG117, pUC19, pBR322, pMW118, pCC1BAC vector, etc. may be used.

For example, a polynucleotide encoding a polypeptide of interest may be inserted into a chromosome through a vector for intracellular chromosome insertion. Insertion of the polynucleotide into the chromosome may be performed by any method known in the art, for example, homologous recombination, but is not limited thereto. The vector may further comprise a selection marker for the confirmation of chromosome insertion. The selection marker is for selecting the cells transformed with vectors, i.e., for confirming the insertion of a nucleic acid molecule of interest, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector comprising a polynucleotide encoding a target polypeptide is introduced into a host cell or a microorganism so that the polypeptide encoded by the polynucleotide may be expressed in the host cell. The transformed polynucleotide may be located regardless of the position, either by being inserted into the chromosome of the microorganism or located outside the chromosome as long as it may be expressed in the host cell. Further, the polynucleotide comprises DNA and RNA encoding a polypeptide of interest. The polynucleotide may be introduced in any form as long as it may be introduced into a host cell and expressed. For example, the polynucleotide may be introduced into a host cell in the form of an expression cassette, which is a gene construct containing all elements required for self-expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

As used herein, the term "operably linked" refers to a constitution of placing a regulatory sequence to an appropriate position to direct expression of a coding sequence. Thus, the term "operably linked" comprises an attachment or linking between a regulatory region of a functional domain having a known or desired activity such as a promoter, a stop codon, a signal sequence, or an enhancer, and a target (gene or polypeptide) such that the expression, secretion, or function of the target may be regulated in accordance with the known or desired activity. For example, the term means that the polynucleotide sequence is functionally linked to a promoter sequence that initiates and mediates transcription of the polynucleotide encoding the desired variant polypeptide of the present disclosure.

As used herein, the term "expression" comprises any process involved in production of a polypeptide, e.g., transcription, post-transcriptional modification, translation, post-translational modification, and secretion, but is not limited thereto.

As used herein, the term "expression vector" refers to a linear or circular nucleic acid molecule comprising a coding sequence and an operably linked regulatory sequence for expression thereof.

As used herein, the term "regulatory sequence" refers to a polynucleotide sequence required for expression of a coding sequence. Each regulatory sequence may be native (from the same gene) or foreign (from different gene) to the coding sequence. Examples of the regulatory sequence may comprise a leader sequence, a polyadenylation sequence, a propeptide sequence, a promoter, a signal peptide sequence, an operator sequence, a ribosome binding site-encoding sequence, and a sequence terminating transcription and translation. A minimum unit of the regulatory sequence may comprise a promoter and a sequence terminating transcription and translation.

With regard to a cell, polynucleotide, polypeptide, or vector, the term "recombinant" used herein refers to the cell, polynucleotide, polypeptide, or vector modified by introduction of a heterologous nucleic acid or polypeptide or alteration of a native polynucleotide or polypeptide, or a cell derived from the modified cell. Thus, for example, a recombinant cell may express a gene that is not found within the native (non-recombinant) form of the cell or may express native genes which are expressed or not expressed, or alternatively, abnormally expressed.

### Host cell

The host cell of the present disclosure may be a microorganism comprising any one or more of the variant of the present disclosure, the polynucleotide of the present disclosure, and the vector comprising the polynucleotide of the present disclosure; a microorganism modified to express the variant of the present disclosure or the polynucleotide of the present disclosure; a microorganism (e.g., recombinant strain) expressing the variant of the present disclosure or the polynucleotide of the present disclosure; or a microorganism (e.g., recombinant strain) having the variant activity of the present disclosure, but is not limited thereto.

### Detailed description of the present disclosure

Hereinafter, specific embodiments of the present disclosure will be described in more detail as follows.

An aspect of the present disclosure provides a serine protease variant.

As used herein, the term "serine protease" refers to an enzyme that belongs to a sub-group of a protease and has a proteolytic activity. Specifically, the serine protease may be an enzyme that degrades proteins by hydrolyzing peptide bonds, and basically has an active serine residue at active sites thereof, and more specifically, may be an enzyme that has a spatial arrangement of amino acid residues of histidine, aspartate, and serine, which may be referred to as a catalytic triad.

The serine protease of the present disclosure may be derived from a microorganism of the genus *Thermobifida* or the genus *Actinorugispora.*

For example, the wild-type of the serine protease provided in the present disclosure may be a serine protease derived from *Thermobifida fusca, Thermobifida cellulosilytica, Thermobifida halotolerans,* or *Actinorugispora endophytica,* for example, a sequence of GenBank: AAZ54522.1, NCBI Accession number WP_016188200.1, GenBank: KUP96625.1, NCBI Reference Sequence: WP_068687914.1, NCBI Reference Sequence: WP_133739400.1, etc.

For example of the wild-type serine protease, the wild-type serine protease of the present disclosure may have an amino acid sequence derived from SEQ ID NO: 8 or SEQ ID NO: 11. For example, the wild-type serine protease may have an amino acid sequence derived from SEQ ID NO: 9 or SEQ ID NO: 12. For example, the wild-type serine protease may be a polypeptide comprising an amino acid sequence represented by SEQ ID NO: 10, but is not limited thereto.

In the present disclosure, the parent sequence of the serine protease, i.e., the serine protease subject to mutation, may comprise, without limitation, not only the wild-type serine protease described above, but also a sequence having the same activity as the amino acid sequence of the wild-type serine protease.

Specifically, the parent sequence of the serine protease may comprise an amino acid represented by any one of SEQ ID NOS: 8 to 12 or an amino acid having at least 75%, 80%, 85%, 90%, 91%, 92%, 93%, 94%, 95%, 96%, 97%, 98%, or 99% or more homology or identity to any one of SEQ ID NOS: 8 to 12. Further, it is apparent that any protein having an amino acid sequence having deletion, modification, substitution, or addition in part of the sequence may also be comprised within the scope of the present disclosure as long as the amino acid sequence has such homology or identity and exhibits efficacy corresponding to that of the protein.

For example, the parent sequence of the protease may comprise an amino acid represented by SEQ ID NO: 6 or 7 or an amino acid having at least 75%, 80%, 85%, 90%, 91%, 92%, 93%, 94%, 95%, 96%, 97%, 98%, or 99% or more homology or identity to SEQ ID NO: 6 or 7.

For another example, the parent sequence of the protease may comprise an amino acid represented by SEQ ID NO: 13, SEQ ID NO: 14, or SEQ ID NO: 15 or an amino acid having at least 75%, 80%, 85%, 90%, 91%, 92%, 93%, 94%, 95%, 96%, 97%, 98%, or 99% or more homology or identity to SEQ ID NO: 13, SEQ ID NO: 14, or SEQ ID NO: 15.

The serine protease variant provided in the present disclosure may be a variant having the serine protease activity, the variant comprising a substitution of an amino acid corresponding to position 180 from the N-terminus of SEQ ID NO: 10 with a different amino acid, in any polypeptide having the serine protease activity or the serine protease.

The serine protease variant of the present disclosure may have the enhanced serine protease enzymatic activity, as compared to the polypeptide before mutation, natural wild-type polypeptide, unmodified polypeptide, or polypeptide of the parent sequence.

For example, the serine protease variant provided in the present disclosure may refer to a variant in which the amino acid corresponding to position 180 of SEQ ID NO: 10 is substituted with a different amino acid as compared to the parent sequence, thereby having enzymatic activity more than 100% of the protein of the parent sequence, among the proteins having the serine protease activity described above.

For example, in the serine protease variant of the present disclosure, phenylalanine (F) which is the amino acid corresponding to position 180 of the amino acid sequence of SEQ ID NO: 10 may be substituted with glycine, alanine, arginine, aspartate, cysteine, glutamic acid, glutamine, histidine, proline, serine, tyrosine, isoleucine, leucine, lysine, tryptophan, valine, methionine, threonine, or asparagine.

For example, in the serine protease variant of the present disclosure, phenylalanine which is the amino acid corresponding to position 180 of the amino acid sequence of SEQ ID NO: 10 may be substituted with tyrosine (Y), glutamic acid (E), histidine (H), leucine (L), methionine (M), asparagine (N), glutamine (Q), or tryptophan (W).

For example, the variant, in which the amino acid corresponding to position 180 from the N-terminus of SEQ ID NO: 10 is substituted with a different amino acid, may comprise an amino acid sequence having at least 75%, 80%, 85%, 90%, 91%, 92%, 93%, 94%, 95%, 96%, 97%, 98%, or 99% or more homology or identity to SEQ ID NO: 7 or 10. For example, the different amino acid may be tyrosine (Y), glutamic acid (E), histidine (H), leucine (L), methionine (M), asparagine (N), glutamine (Q), or tryptophan (W).

Meanwhile, a mature region of GenBank: AAZ54522.1 (SEQ ID NO: 8) corresponds to SEQ ID NO: 9, a mature region of NCBI Reference Sequence WP_016188200.1 (SEQ ID NO: 11) corresponds to SEQ ID NO: 12 of the present disclosure, and the sequence excluding the signal peptide from SEQ ID NO: 9 and SEQ ID NO: 12 corresponds to SEQ ID NO: 10 of the present disclosure.

Further, SEQ ID NO: 7 has improved enzymatic activity, as compared to the polypeptide of SEQ ID NO: 10, because the amino acids corresponding to positions 12 and 116 of SEQ ID NO: 10 are substituted with different amino acids.

Further, it is obvious that the serine protease variant of the present disclosure may comprise deletions or additions of amino acids that have minimal effect on the properties and secondary structure of the serine protease in which the amino acid corresponding to position 180 of SEQ ID NO: 10 is substituted with a different amino acid as described. In addition, through sequence alignment known in the art, those skilled in the art may recognize that position 180 from the N-terminus of SEQ ID NO: 7 of the present disclosure corresponds to position 180 of SEQ ID NOS: 10, 13 to 15, position 331 of SEQ ID NOS: 6, 9, and 12, and position 180 of SEQ ID NOS: 10, 13 to 15, and position 361 of SEQ ID NOS: 8 and 11, and SEQ ID NO: 7 is comprised in SEQ ID NO: 6, and SEQ ID NO: 10 is comprised in SEQ ID NOS: 8, 9, 11, and 12.

Accordingly, the serine protease variant of the present disclosure may be exemplified by a variant in which the amino acid corresponding to position 180 of SEQ ID NO: 10 (the amino acid at position 331 of SEQ ID NO: 6 and the amino acid at position 361 of SEQ ID NO: 8) is substituted, in connection with SEQ ID NO: 6 and SEQ ID NO: 8. The serine protease of the present disclosure may be also exemplified by a variant in which the amino acid corresponding to position 180 of SEQ ID NO: 10 (the amino acid at position 331 of SEQ ID NO: 9) is substituted, in connection with SEQ ID NO: 9.

Further, in the present disclosure, the description of the amino acid at position 180 of SEQ ID NO: 10 may also be applied to the amino acid at position 180 of SEQ ID NOS: 7, 13 to 15, the amino acid at position 331 of SEQ ID NOS: 6 and 9, and the amino acid at position 361 of SEQ ID NO: 8.

Similarly, the description of the amino acids at position 12 and 116 of SEQ ID NO: 10 may also be applied to the amino acids at positions 12 and 116 of SEQ ID NO: 7, the amino acids at positions 163 and 267 of SEQ ID NOS: 6, 9 and 12, and the amino acids at positions 193 and 297 of SEQ ID NO: 8 and SEQ ID NO: 11.

For example, the serine protease variant of the present disclosure may comprise an amino acid sequence in which the amino acid corresponding to the amino acid at position 180 of SEQ ID NO: 10 is substituted with a different amino acid and may have at least 75% or more and less than 100% sequence homology to SEQ ID NO: 6. In another embodiment, the serine protease variant of the present disclosure may comprise an amino acid sequence in which the amino acid corresponding to the amino acid at position 180 of SEQ ID NO: 10 is substituted with a different amino acid and may have 75% or more and less than 100% sequence homology to SEQ ID NO: 8.

For example, the serine protease variant of the present disclosure may comprise an amino acid sequence in which the amino acid(s) corresponding to the amino acid(s) at position 12 and/or at position 116 from the N-terminus of SEQ ID NO: 10 are/is substituted with a different amino acid.

Specifically, in the serine protease variant of the present disclosure, phenylalanine at position 12 of the amino acid sequence of SEQ ID NO: 10 may be substituted with glycine, alanine, arginine, aspartate, cysteine, glutamic acid (glutamate), asparagine, glutamine, histidine, proline, serine, tyrosine, isoleucine, leucine, lysine, tryptophan, valine, methionine, or threonine, and/or asparagine at position 116 may be substituted with glycine, alanine, arginine, aspartate, cysteine, glutamic acid, glutamine, histidine, proline, serine, tyrosine, isoleucine, leucine, lysine, phenylalanine, tryptophan, valine, methionine, or threonine, but is not limited thereto.

Specifically, the variant may be a protein in which the amino acid corresponding to position 12 of the amino acid sequence of SEQ ID NO: 10 may be substituted with tyrosine (Y), serine (S), alanine (A), or arginine (R), or the amino acid corresponding to position 116 may be substituted with aspartate (D), serine (S), threonine (T), or glycine (G), or the amino acids corresponding to positions 12 and 116 of the amino acid sequence of SEQ ID NO: 10 may be each substituted with tyrosine (Y) and aspartate (D); tyrosine (Y) and serine (S); serine (S) and aspartate (D); serine (S) and threonine (T); or alanine (A) and glycine (G).

For example, in the serine protease variant of the present disclosure, (i) the amino acid corresponding to position 12 of SEQ ID NO: 10 may be tyrosine (Y), alanine (A), serine (S), or arginine (R); (ii) the amino acid corresponding to position 116 of SEQ ID NO: 10 may be aspartate (D), serine (S), threonine (T), or glycine (G); or (iii) the amino acid corresponding to position 12 of SEQ ID NO: 10 may be tyrosine (Y), alanine (A), serine (S) or arginine (R), and the amino acid corresponding to position 116 of SEQ ID NO: 10 may be aspartate (D), serine (S), threonine (T), or glycine (G). For example, in the serine protease variant of the present disclosure, the amino acid corresponding to position 12 of SEQ ID NO: 10 may be tyrosine, and the amino acid corresponding to position 116 of SEQ ID NO: 10 may be aspartate.

For example, in the serine protease variant of the present disclosure, (i) the amino acid corresponding to position 12 of SEQ ID NO: 10 may be phenylalanine (F); (ii) the amino acid corresponding to position 116 of SEQ ID NO: 10 may be asparagine (N); or (iii) the amino acid corresponding to position 12 of SEQ ID NO: 10 may be phenylalanine (F), and the amino acid corresponding to position 116 may be asparagine (N). For example, in the serine protease variant of the present disclosure, the amino acid corresponding to position 12 of SEQ ID NO: 10 may be phenylalanine, and the amino acid corresponding to position 116 may be asparagine.

Another aspect of the present disclosure provides a polynucleotide encoding the serine protease variant of the present disclosure.

The polynucleotide encoding the serine protease variant of the present disclosure may comprise any polynucleotide sequence without limitation, as long as it encodes the serine protease variant having the enhanced activity of the present disclosure.

The polynucleotide of the present disclosure may undergo various modifications in the coding region without changing the amino acid sequence of the polypeptide, due to codon degeneracy or in consideration of the codons preferred in an organism in which the polypeptide is to be expressed. Specifically, the polynucleotide may comprise any polynucleotide sequence without limitation, as long as it is a polynucleotide sequence encoding a variant in which the amino acid corresponding to position 180 from the N-terminus of SEQ ID NO: 10 is substituted with a different amino acid.

Further, as described above, the serine protease variant of the present disclosure comprises a variant in which the amino acid corresponding to position 180 from the N-terminus of SEQ ID NO: 10, i.e., the amino acid corresponding to position 180 from the N-terminus of SEQ ID NO: 10 (the amino acid at position 331 of SEQ ID NO: 9 or 12, the amino acid at position 361 of SEQ ID NO: 8 or 11) is substituted, in connection with SEQ ID NOS: 8, 9, 11, or 12, which is an amino acid sequence comprising SEQ ID NO: 10, it is thus obvious that a polynucleotide sequence encoding such a serine protease variant is also comprised in the scope of the present disclosure.

Further, a probe which may be prepared from a known nucleotide sequence, for example, any polynucleotide sequence which hybridizes with a sequence complementary to the entirety or a part of the nucleotide sequence under stringent conditions to encode a protein having the activity of the variant in which the amino acid corresponding to position 180 from the N-terminus of SEQ ID NO: 10 is substituted with a different amino acid, may be comprised without limitation.

For example, the polynucleotide sequence encoding the serine protease variant of the present disclosure may comprise a sequence encoding an amino acid sequence from position 152 to position 338 of an amino acid sequence encoded by SEQ ID NO: 16, in which a codon encoding the amino acid corresponding to the amino acid at position 361 of the amino acid sequence encoded by the polynucleotide sequence of SEQ ID NO: 16 is substituted with an amino acid other than phenylalanine, for example, tyrosine (Y), glutamic acid (E), histidine (H), leucine (L), methionine (M), asparagine (N), glutamine (Q), or tryptophan (W).

Still another aspect of the present disclosure provides a vector comprising the polynucleotide encoding the serine protease variant of the present disclosure.

Still another aspect of the present disclosure provides a microorganism comprising any one or more of the serine protease variant, the polynucleotide encoding the same, and the vector comprising the same.

The microorganism comprising any one or more of the serine protease variant, the polynucleotide encoding the variant, and the vector comprising the same may be specifically a microorganism prepared by transforming with the vector comprising the polynucleotide encoding the variant, but is not limited thereto.

The microorganism may be a microorganism expressing the serine protease variant.

As used herein, the term "protein allowed to be expressed/to be expressed/expressed" refers to a state in which the target protein is introduced into the microorganism or is modified to be expressed in the microorganism. With respect to the objects of the present disclosure, the "target protein" may be the serine protease variant described above.

Specifically, "introduction of the protein" means exhibiting activity of a particular protein that was not originally possessed by a microorganism, or exhibiting enhanced activity, as compared with the endogenous activity of the corresponding protein or the activity before modification. For example, a polynucleotide encoding a particular protein may be introduced into a chromosome in a microorganism or a vector containing a polynucleotide encoding a particular protein may be introduced into a microorganism to exhibit its activity.

The microorganism may be a recombinant microorganism, wherein the recombination may be achieved by genetic modification such as transformation.

The recombinant microorganism may have the enhanced serine protease activity of the present disclosure.

The "enhancement of activity" may mean that activity is improved, as compared with the endogenous activity of a specific protein possessed by a microorganism or activity before modification. The "endogenous activity" may mean the activity of a specific protein originally possessed by a parent strain before the trait is changed, when the trait of the microorganism is changed by genetic variation due to natural or artificial factors.

Specifically, the enhancement of activity of the protein variant in the present disclosure may be achieved by any one or more methods of a method of increasing the intracellular copy number of the gene encoding the protein variant, a method of introducing a variation into the expression regulatory sequence of the gene encoding the protein variant, a method of replacing the expression regulatory sequence of the gene encoding the protein variant by a sequence having strong activity, a method of replacing a gene encoding a natural protein having the serine protease activity on the chromosome with the gene encoding the protein variant, and a method of additionally introducing a variation into the gene encoding the variant to enhance the activity of the protein variant, but is not limited thereto.

Next, the modification of the expression regulatory sequence for increasing expression of a polynucleotide may be, but is not particularly limited to, performed by inducing a variation in the sequence via deletion, insertion, non-conservative or conservative substitution, or a combination thereof so as to further enhance the activity of the expression regulatory sequence, or by replacing the nucleotide sequence with a nucleotide sequence with a stronger activity. The expression regulatory sequence may comprise, but is not particularly limited to, a promoter, an operator sequence, a sequence encoding a ribosome-binding domain, and a sequence regulating termination of transcription and translation, etc.

A strong promoter, instead of the original promoter, may be connected to the upstream region of the expression unit of the polynucleotide, but is not limited thereto. Examples of the known strong promoter may comprise cj1 to cj7 promoters (Korean Patent No. 10-0620092), lac promoter, trp promoter, trc promoter, tac promoter, lamda phage PR promoter, PL promoter, tet promoter, gapA promoter, SPL7 promoter, SPL13(sm3) promoter (Korean Patent No. 10-1783170), O2 promoter (Korean Patent No. 10-1632642), tkt promoter, and yccA promoter, etc., are is not limited thereto.

Furthermore, modification of a polynucleotide sequence on chromosome may be, but is not particularly limited to, performed by inducing a variation on the expression regulatory sequence via deletion, insertion, non-conservative or conservative substitution of the nucleotide sequence, or a combination thereof so as to further enhance the activity of the polynucleotide sequence, or by replacing the polynucleotide sequence with a polynucleotide sequence which is improved to have a stronger activity.

Such introduction and enhancement of the protein activity may increase the activity or concentration of the corresponding protein from at least 1%, 10%, 25%, 50%, 75%, 100%, 150%, 200%, 300%, 400% or 500%, to a maximum of 1000% or 2000%, relative to the activity or concentration of a protein of a wild-type or unmodified microbial strain, but is not limited thereto.

The host cell or microorganism of the present disclosure may be any microorganism as long as it expresses the serine protease variant by comprising the polynucleotide of the present disclosure or the vector of the present disclosure.

Specifically, the microorganism may be a microbial strain belonging to the genus *Escherichia,* the genus *Serratia,* the genus *Erwinia,* the genus *Enterobacteria,* the genus *Providencia,* the genus *Salmonella,* the genus *Streptomyces,* the genus *Pseudomonas,* the genus *Brevibacterium,* the genus *Corynebacterium,* or the genus *Bacillus,* etc., and specifically, a strain of *Bacillus subtilis, Bacillus licheniformis, Bacillus amyloliquefaciens, Bacillus velezensis, Escherichia coli, Corynebacterium glutamicum, Aspergillusoryzae,* etc., and more specifically, *Bacillus subtilis,* but is not limited thereto.

Still another aspect of the present disclosure provides a composition comprising any one or more of the serine protease variant of the present disclosure; and a microorganism expressing the same.

The serine protease variant to be comprised in the composition of the present disclosure may comprise the microorganism itself expressing the serine protease variant or may be in a form isolated and purified from the microorganism expressing the serine protease variant, but is not limited thereto.

The composition of the present disclosure may further comprise any components suitable for application in various industrial fields. Examples of substances to be added comprise stabilizers, surfactants, builders, chelating agents, dispersants, enzymes, enzyme stabilizers, catalysts, activators, carriers, mixtures, lubricants, disintegrants, excipients, solubilizers, suspending agents, colorants, flavors, buffers, preservatives, analgesics, solubilizers, isotonic agents, stabilizers, diluents, lubricants, preservatives, etc., but are not limited thereto.

For example, the composition provided in the present disclosure may further comprise naturally occurring substances or non-naturally occurring substances, in addition to the variants provided in the present disclosure.

For example, the composition according to the present disclosure may be a feed composition.

The "feed composition" of the present disclosure refers to any natural or artificial diet, one meal or the like, or an ingredient of the one meal for animals to eat, intake, and digest or being suitable therefor, and the feed may be prepared in various forms known in the art.

The feed composition may be a feed additive.

The kind of the feed is not particularly limited, and feeds commonly used in the art may be used. Non-limiting examples of the feed may comprise vegetable feeds such as grains, roots/fruits, food processing by-products, algae, fibers, pharmaceutical by-products, oils and fats, starches, gourds, and grain by-products; and animal feeds such as proteins, inorganic substances, oils and fats, minerals, oils and fats, single-cell proteins, animal planktons, or foods. These feeds may be used alone or in combination of two or more thereof.

The feed composition of the present disclosure may further comprise one or more selected from organic acids such as citric acid, fumaric acid, adipic acid, lactic acid, malic acid, etc.; phosphates such as sodium phosphate, potassium phosphate, acid pyrophosphate, polyphosphate (polymerized phosphate), etc.; or natural antioxidants such as polyphenol, catechin, alpha-tocopherol, rosemary extract, vitamin C, green tea extract, licorice root extract, chitosan, tannic acid, phytic acid, etc.

The feed composition of the present disclosure may further comprise one or more selected from adjuvant ingredients such as amino acids, minerals, vitamins, antibiotics, antibacterial substances, antioxidants, antifungal enzymes, and microbial preparations in other probiotic forms; grains, e.g., pulverized or crushed wheat, oat, barley, corn, and rice; vegetable protein feeds, e.g., rapeseed, beans, and sunflowers as a main ingredient; animal protein feeds, e.g., powders of blood, meat, bone, and fish; sugar and dairy products, e.g., dry ingredients formed of various kinds of dry milk and whey powder; lipids, e.g., main ingredients such as animal fats and vegetable oils arbitrarily liquefied by heating; and additives such as nutritional supplements, digestion and absorption promoters, growth promoters, and prophylactic agents, etc.

The feed composition of the present disclosure may be in the form of a dry or liquid preparation, and may further comprise an excipient for feed. The excipient for feed may be, for example, zeolite, corn flour, and rice bran, but is not limited thereto.

The feed composition of the present disclosure may further comprise an enzyme preparation in addition to the serine protease variant. For example, the feed composition may further comprise one or more selected from a lipid degrading enzyme such as lipase, phytase that degrades phytic acid into a phosphate and inositol phosphate, amylase that is an enzyme catalyzing the hydrolysis of α-1,4-glycoside bonds comprised in starch, glycogen, etc., phosphatase that is an enzyme catalyzing the hydrolysis of organophosphate esters, maltase that catalyzes the hydrolysis of maltose into two glucose molecules, and a converting enzyme that catalyzes the hydrolysis of saccharose into a glucose-fructose mixture, etc., but is not limited thereto.

The feed composition of the present disclosure may be administered to animals singly or in combination with other feed additives comprised in an edible carrier. In addition, the feed composition may be easily administered as a feed additive or top dressing by being directly mixed in a livestock feed or separately from the feed, or in a separate oral formulation or in combination with other ingredients. In addition, once-daily dose or multiple-divided daily dose may be used, as commonly known in the art.

Examples of the animal to which the feed composition of the present disclosure is applied may comprise livestock such as beef cattle, dairy cattle, calves, pigs, piglets, sheep, goats, horses, rabbits, dogs, cats, etc.; and poultry such as chicks, hens, domestic chickens, roosters, ducks, geese, turkeys, quails, small birds, etc., but are not limited thereto.

The amount of the serine protease variant of the present disclosure which is comprised in the feed composition of the present disclosure is not particularly limited, and may be appropriately adjusted according to the purpose thereof. In an embodiment, the serine protease variant may be comprised in an appropriate amount for degrading a protein source substance while surviving in the digestive tract of livestock for a long period of time as commonly known in the art to which the present disclosure pertains, but is not limited thereto.

For example, the composition according to the present disclosure may be a food composition.

The protease protein may be used in a liquid or solid food composition. In addition, the food may be powders, pills, beverage, tea, or additives for general foods.

In an embodiment, the food may be a group of foods requiring a protease, such as dairy products, health functional foods for improving bowel movement and weight loss, and health functional foods for preventing hypertension, etc.

In another embodiment, the protease variant may be comprised in various food compositions as a food solubilizer, food softening agent, and meat modifier. In various other embodiments, the serine protease variant may be added during baking to be used in a step for gluten network breakdown. Alternatively, the serine protease variant may be used to catalyze the hydrolysis of food proteins (e.g., proteins in milk). Alternatively, the serine protease variant may be comprised in various food compositions for the purpose of rendering, preparing flavoring agents, reducing bitterness, changing emulsifying properties, producing bioactive peptides, reducing allergy-causing antigens in proteins, etc. However, this is only an illustrative embodiment and the uses thereof are not limited thereto.

The amount of the serine protease variant in the food composition may be appropriately adjusted by those skilled in the art according to the purpose thereof.

For example, the composition according to the present disclosure may be a detergent composition.

The detergent composition according to the present disclosure may be in the form of first and second aqueous detergent compositions, non-aqueous liquid detergent compositions, cast solids, granules, particles, compressed tablets, gels, pastes, or slurries. The detergent composition may be used to remove stubborn food stains, food residue films, and other small amounts of food compositions.

The detergent composition according to the present disclosure may be provided in the form of a detergent composition for cleaning hard surfaces, a detergent composition for cleaning fabrics, a detergent composition for dishwashing, a detergent composition for oral cleaning, a detergent for cleaning dentures, or a contact lens cleaning solution, but is not limited thereto.

For example, the composition according to the present disclosure may be a pharmaceutical composition.

The pharmaceutical composition of the present disclosure may be used as a pharmaceutical composition for digestive enzymes to improve digestive diseases, digestive disorders, and abnormalities after digestive surgery, a thrombolytic or antithrombotic composition directly applied to blood clots to dissolve fibrin, an anti-inflammatory drug acting as an *in vivo* defense system to remove inflammatory substances or necrotic tissue, or an anti-inflammatory drug to alleviate edema after surgery or wounds.

The pharmaceutical composition may further comprise a pharmaceutically acceptable or nutritionally acceptable carrier, excipient, diluent, or accessory ingredient according to the methods or purposes of use. The carrier, excipient, or diluent may comprise one or more selected from the group consisting of lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxy benzoate, propyl hydroxy benzoate, talc, magnesium stearate and mineral oils, dextrin, calcium carbonate, propylene glycol, liquid paraffin, and physiological saline solution, but is not limited thereto.

The serine protease variant of the present disclosure or the microorganism expressing the same may also be used for the purpose of producing cosmetics, processing leather, preparing pharmaceuticals, preparing diagnostic agents, managing wastes, and preparing chemicals for academic research, in addition to the above-described uses.

However, the uses are exemplarily described, and the serine protease variant may also be used for any other purposes of denaturing, degrading, or removing proteinaceous substances known in the art.

Still another aspect of the present disclosure provides a method of preparing the serine protease variant, the method comprising the step of culturing the microorganism comprising the serine protease variant of the present disclosure, the polynucleotide encoding the variant, or the vector comprising the polynucleotide.

For example, the method of preparing the serine protease variant may further comprise the step of collecting the variant polypeptide having the serine protease activity of the present disclosure which is expressed in the step of culturing the microorganism.

In another embodiment, the variant which is expressed in the step of culturing the microorganism may not be collected. The microorganism itself expressing the variant may be used as a source of the variant.

Still another aspect of the present disclosure provides a method of degrading casein using the serine protease variant. Example of the degradation product may comprise tyrosine.

Casein may be degraded using the variant of the present disclosure, a host cell expressing the variant, and a composition comprising the variant and/or host cell. In this degradation step, cofactors, coenzymes, etc. may be added together. The step of hydrolyzing a substrate may be performed under optimal pH and temperature conditions, etc., and those skilled in the art may select appropriate conditions.

### [Mode for Carrying Out the Invention]

Hereinafter, the present disclosure will be described in more detail with reference to Examples and Experimental Example. However, these Examples and Experimental Example are only for illustrating the present disclosure, and the scope of the present disclosure is not intended to be limited by these Examples and Experimental Example.

### Example 1. Selection of Thermobifida fusca-derived Serine Protease Variant

### Example 1-1. Preparation of Thermobifida fusca-derived Serine Protease Library

A random mutation was introduced into a gene encoding a region corresponding to a mature region of a *Thermobifida* fusca-derived serine protease by error-prone PCR. Variant sequences (F12Y, N116D) licensed in prior patents were as a PCR template in this study (PCT/KR2020/016775). The error-prone PCR was performed using a Diversify^{™} PCR Random Mutagenesis Kit (Clontech, Cat# 630703), and the PCR conditions are as described in Table 1. It was confirmed that the mutation was introduced at a frequency of 6.2 mutations/kb under the conditions below.

**[Table 1]**

| Composition | | Amount | Remarks |
|---|---|---|---|
| Template DNA | | 0.5 ng | SEQ ID NO: 1 |
| Forward primer | | 5 uM | SEQ ID NO: 2 |
| Reverse primer | | 5 uM | SEQ ID NO: 3 |
| 10X Titanium Taq buffer | | 5 uL | |
| MnSO4 | | 640 uM | |
| dGTP | | 40 uM | |
| 50X Diversify dNTP mix | | 1 uL | |
| 50X dNTP mix | | 1 uL | |
| Titanium Taq Polymerase | | 1 uL | |
| Total | | 50 uL | Adjust to 50ul with DW |

| PCR conditions | | | |
|---|---|---|---|
| I. | 94°C | 30 sec | Repeat 50 cycles of II and III |
| II. | 94°C | 30 sec | |
| III. | 68°C | 1 min | |
| IV. | 68°C | 1 min | |
| V. | 4°C | ∞ | |

PCR fragments obtained through the process were ligated to vectors amplified using primers presented in Table 2 using an In-FusionR HD cloning kit (Clontech) and transformed into DH5α cells to obtain colonies. The plasmids were purified from the obtained colonies to obtain libraries having a size of about 5*10⁴.

**[Table 2]**

| | |
|---|---|
| Template DNA(pBE-S-TFP) | SEQ ID NO: 1 |
| Forward primer | SEQ ID NO: 4 |
| Reverse primer | SEQ ID NO: 5 |

### Example 1-2. Screening of Thermobifida fusca-derived Serine Protease Library

*A Bacillus subtilis* LB700 strain, which easily released proteins, was transformed with the protease library prepared in Example 1-1, and screening was performed. The screening was performed by a two-stage process. In a first stage, the *Bacillus subtilis* strain transformed with the library was plated on a 2% skim milk plate, and colonies were selected based on the formed halo size. The transformation of *Bacillus subtilis* was performed according to the Groningen method, and the composition of the skim milk plate used in the screening is as presented in Table 3.

**[Table 3]**

| Composition | Amount | Remarks |
|---|---|---|
| M9 minimal salts | 11.28 g | BD, cat#248510 |
| Agar | 20 g | |
| Skim milk | 20 g | BD, cat#232100 |
| 50%Glucose | 20 g | |
| 1M MgSO4 | 2 mM | |
| 1M CaCl2 | 0.1 mM | |
| Kanamycin | 50 ug/ml | |

| | | |
|---|---|---|
| (based on 1 L) | | |

A second stage relates to a method of selecting the colonies, which were primarily selected in the first stage, by azocasein color development. A Brain Heart Infusion (BHI, bd, cat#53286) liquid medium containing a kanamycin antibiotic was placed in a 96-deep-well plate, and the colonies selected in the first stage were inoculated thereinto, followed by culturing at 37°C for 20 hours to 24 hours. After the culturing, a supernatant comprising an enzyme was obtained by centrifugation, and the supernatant was mixed with an equal amount of 2% (w/v) azocasein as a substrate, and allowed to react at 37°C for 1 hour. The reaction was terminated by adding a 3-fold volume of 10% trichloro acetic acid (TCA) to the enzyme reaction solution, and coagulated proteins were removed by centrifugation. The color development reaction was performed by mixing with an equal amount of NaOH, and then the absorbance at 440 nm was measured to compare the degree of color development. Through this process, colonies having an increase in absorbance by 130% or more compared to that of the serine protease of SEQ ID NO: 6 were selected.

### Example 2. Preparation of Selected Variant and Evaluation of Activity

### Example 2-1: Preparation of Variant

As a result of analyzing the sequences of variants selected through screening, it was confirmed that, based on SEQ ID NO: 10, position 180 (phenylalanine, Phe) was further substituted with tyrosine(Tyr), in addition to F12Y, N116D mutations.

### Example 2-2. Evaluation of Activity

In this study, the protease activity was determined by measuring the amount of tyrosine dissociated from the substrate through an enzymatic reaction using casein (from bovine milk) as a substrate. *Bacillus subtilis* LB700 strain was transformed with the obtained F12Y/N116D/F180Y variant, and inoculated into BHI medium containing kanamycin antibiotic and cultured at 37°C for 20 hours to 24 hours, and a portion of the culture medium was used for enzyme reaction. The culture solution (enzyme) diluted in 0.1 mL of 50 mM sodium borate (pH 10.5 at room temperature) buffer solution and 0.1 mL of 1.0% casein as the substrate (Sigma-Aldrich Co.) were mixed and allowed to react at 40°C for 10 minutes. Thereafter, the reaction was terminated by adding 0.2 mL of trichloroacetic acid (TCA, Sigma-Aldrich Co.) solution. After centrifugation (10,000 X g, 10 min), to 0.15 mL of supernatant, 0.75 mL of 0.4 M sodium carbonate anhydrous and 0.15 mL of Folin Ciocalteu (Sigma-Aldrich Co.) solution diluted 3-fold were added, and color development was allowed at 40°C for 20 minutes, and absorbance at 680 nm was measured using a spectrophotometer. Protease activity was calculated from a standard curve quantifying tyrosine (Sigma-Aldrich Co.), and the activity unit was defined as the amount of enzyme that produces 1 µg of tyrosine per minute. The activity thus measured is shown in Table 4.

**[Table 4]**

| | Enzyme activity (unit/ml) |
|---|---|
| F12Y/N116D | 142 |
| F12Y/N116D/F180Y | 312 |

As a result of the measurement, it was confirmed that when the F180Y mutation was further added, the activity increased approximately 2.2 times under conditions of pH 10.5 and 40°C, compared to the F12Y/N116D variant.

### Example 3. Preparation of Residue 180 Mutant and Evaluation of Activity

The results of Example 2 confirmed that position 180 of SEQ ID NO: 10 was effective in improving the serine protease activity, and additionally, saturation mutagenesis and introduction of F180Y based on the wild-type sequence (SEQ ID NO: 10) were performed.

**[Table 5]**

| | |
|---|---|
| Template DNA | SEQ ID NO: 1 |
| Forward primer | SEQ ID NOS: 17, 19, 21, 23, 25, 27, 29, 31 |
| Reverse primer | SEQ ID NOS: 18, 20, 22, 24, 26, 28, 30, 32 |

The activities of the obtained variants were evaluated using the same method as described in Example 2-2.

The experimental results are shown in Table 6 below.

**[Table 6]**

| Variant | Enzyme activity (Unit/ml) | Relative enzyme activity ratio (%) |
|---|---|---|
| Wild-type | 94 | 100 |
| F180Y | 141 | 150 |
| F12Y/N116D/**F180Y** | 276 | 294 |
| F12Y/N116D/**F180E** | 154 | 164 |
| F12Y/N116D/**F180H** | 167 | 178 |
| F12Y/N116D/**F180L** | 124 | 132 |
| F12Y/N116D/**F180M** | 174 | 185 |
| F12Y/N116D/**F180N** | 152 | 162 |
| F12Y/N116D/**F180Q** | 131 | 139 |
| F12Y/N116D/**F180W** | 234 | 249 |

It was confirmed that the activity was improved by introducing the mutation at position 180 as described.

### Example 4. Evaluation of Activity after Introduction of Mutation into Position Corresponding to Position 180 in Various Serine Proteases

Activity was evaluated by changing the parent sequence and introducing a substitution into the amino acid corresponding to position 180 of SEQ ID NO: 10. In the experiment, serine protease derived from *Thermobifida halotolerans* (SEQ ID NO: 13) and serine protease derived from *Actinorugispora endophytica* (SEQ ID NO: 14) were used.

The results of homology comparison between each sequence and SEQ ID NO: 10 and introduction of F180Y mutation are shown in the table below.

**[Table 7]**

| Origin | Variant | Enzyme activity (Unit/ml) | Relative enzyme activity ratio | Homology % |
|---|---|---|---|---|
| *Thermobifida halotolerans* | Wild-type | 111 | 100% | 81.8 % |
| | F180Y | 133 | 120% | |
| *Actinorugispora endophytica* | Wild-type | 421 | 100% | 81.3 % |
| | F180Y | 651 | 155% | |

As a result, it was confirmed that the activity was greatly improved by substituting the amino acid residue at position 180 with Y.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A serine protease variant, wherein an amino acid corresponding to position 180 of SEQ ID NO: 10 is substituted with a different amino acid.

2. The serine protease variant of claim 1, wherein the different amino acid is tyrosine (Y), glutamic acid (E), histidine (H), leucine (L), methionine (M), asparagine (N), glutamine (Q), or tryptophan (W).

3. A serine protease variant, wherein an amino acid corresponding to position 331 of SEQ ID NO: 9 is substituted with a different amino acid.

4. A serine protease variant, wherein an amino acid corresponding to position 361 of SEQ ID NO: 8 is substituted with a different amino acid.

5. The serine protease variant of claim 1, wherein the variant has 75% or more homology or identity with an amino acid sequence of SEQ ID NO: 10.

6. A polynucleotide encoding the serine protease variant of any one of claims 1 to 5.

7. A vector comprising the polynucleotide of claim 6.

8. A microorganism comprising the serine protease variant of any one of claims 1 to 5, a polynucleotide encoding the variant, or a vector comprising the polynucleotide.

9. A composition comprising any one or more of the serine protease variant of any one of claims 1 to 5; and
a microorganism comprising the serine protease variant, a polynucleotide encoding the variant, or a vector comprising the polynucleotide.

10. The composition of claim 9, wherein the composition is a feed composition.
